# EUROPEAN PATENT SPECIFICATION

(11) **EP 3 231 390 B1**
(45) Date of publication and mention of the grant of the patent: **04.12.2019**
(21) Application number: 17166495.6
(22) Date of filing: 13.04.2017
(51) Int. Cl.: A61B 90/30, F21V 21/15

(54) **OPERATING ROOM LIGHTHEAD AND METHOD FOR PRESENTING ILLUMINATION ADJUSTMENT INSTRUCTIONS TO AN OPERATOR OF THE OPERATING ROOM LIGHTING SYSTEM**
OPERATIONSSAALLAMPENKOPF UND VERFAHREN ZUR PRÄSENTATION VON BELEUCHTUNGSEINSTELLUNGSANWEISUNGEN AN EINEN BEDIENER DES OPERATIONSRAUMBELEUCHTUNGSSYSTEMS
TÊTE D'ÉCLAIRAGE DE SALLE D'OPÉRATION ET PROCÉDÉ DE PRÉSENTATION DES INSTRUCTIONS DE RÉGLAGE D'ÉCLAIRAGE À UN OPÉRATEUR DU SYSTÈME D'ÉCLAIRAGE DE SALLE D'OPÉRATION

(30) Priority: 15.04.2016 FI 20165333
(43) Date of publication of application: 18.10.2017
(73) Proprietor: Merivaara Oy, 15150 Lahti (FI)
(72) Inventor: BÄRLUND, Paul, 15170 Lahti (FI)
(74) Representative: Berggren Oy, Helsinki & Oulu

(56) References cited:
- EP-A2- 2 618 042
- WO-A1-2015/049866
- US-A1- 2015 366 036
- US-B1- 6 633 328

## Description

### TECHNICAL FIELD

The present invention relates generally to lighting technology and lighting methods. In particular, the invention relates to lighting solutions, wherein the operator is able to modify a desired light field feature by means of a purpose-designed user interface while operating the lighting solution.

### BACKGROUND

A lighthead can be generally provided with various user interface elements such as pushbuttons or other adjustment elements for a desired light field feature, such as the light field intensity, to be adjusted personally by the lighthead operator. It has been possible to accompany the elements, e.g. by methods of printing technology, with operating instructions such as a graphic indication concerning a feature adjustable with the elements, e.g. the text 'light field intensity', and another indication about the actual effect on said feature resulting from operating the elements. For example, the symbolic instruction '+', provided in connection with a pushbutton, indicates to the operator that the adjustable feature increases in value by pushing the button while the symbol '-' indicates respectively a decrease of the feature in value, typically by the amount of a predetermined numerical value.

In some working situations, such conventional adjustment elements, and especially instructions, have nevertheless proved inconvenient or at least non-optimal. In a medical working environment, such as in an operating or examination room environment, it is impractical from the standpoint of a person performing a treatment procedure or diagnosis to take his/her eyes off an object being treated or diagnosed, e.g. human tissue or more generally a patient, for conducting a desired adjustment. Particularly during performance of an operating room procedure, it may also be hazardous if the surgeon, for example in the middle of a surgical operation, while adjusting the lighting, would have to take his/her eyes off the surgical site.

In practice, however, it is most often necessary to disengage one's eyes, because it is highly inconvenient to operate the adjustment element just by heart and by so-called feeling around with the hand for first finding a correct adjustment element, i.e. a user input element such as for example a pushbutton, and then for performing the actual adjustment measure. Therefore, in practice, the adjustment stops the performance of an actual procedure at least for a while. Likewise, because the working of an adjustment element, such as for example a sterile handle of the operating room lighthead, wherein the adjustment is conducted with buttons on the handle having accompanying adjustment instructions provided by way of graphic symbols, requires that the eyes be focused on the lighthead itself, there is no way of observing adjustment results without turning one's eyes back to a site of illumination, leading easily to a situation in which it is necessary, by shifting one's eyes back and forth, to alternately conduct adjustment measures and to check the adjustment result from the site in order to establish a desired final value for the feature being adjusted.

Problems of similar nature are also encountered in other working situations and environments, wherein a dynamic adjustment of lighting would be desirable in connection with on-going work. These include tasks that require precision and focusing, such as for example jobs for micromechanics, watchmakers or in other precision industries.

EP2618042 discloses a lighting system comprising a lighthead for an operating theater, wherein the lighthead comprises two groups of mounting supports disposed so that the mounting supports are apart, the mounting supports being provided with a plurality of light elements. The mounting supports may be movable to have a direction of the mounting supports remain constant with respect to an airflow arriving at the lighthead, keeping the airflow laminar.

### SUMMARY

It is an objective of the invention to at least alleviate one or more shortcomings of prior art solutions in relation to lighting fixtures.

The objective is attained with various embodiments for a lighting apparatus and a lighting method of the invention.

An operating room lighthead according to one aspect of the invention comprises a plurality of light sources for generating a light field on a surgical site to be illuminated on a patient, as well as adjustment elements for modifying features of said light field. The adjustment elements comprise a user interface for modifying features of the light field by the actions of a lighthead operator, the lighthead being characterized in that the user interface further comprises projection elements for projecting, around a light field, adjustment instructions relating to a feature of the lighthead-generated light field, and the lighthead is further provided with at least one laser light source specifically for projecting said adjustment instructions. The projection elements comprise a light-transmitting optical film which is adapted to produce an adjustment instruction from light generated by said at least one laser light source, said film comprising diffractive and/or refractive optical microstructures for producing adjustment instructions.

The invention relates particularly to a lighthead of claim 1, wherein the lighthead has its light field focusable on a site of a patient subjected to a medical procedure.

A method according to another aspect of the invention for presenting illumination adjustment instructions to an operator of an operating room lighthead, said lighthead including a plurality of light sources for generating a light field on a surgical site on a patient, as well as adjustment elements for modifying features of said light field, said adjustment elements being provided with a user interface for modifying features of the lighthead-generated light field by the actions of a lighthead operator, is characterized in that the method comprises projecting adjustment instructions for modifying features of a lighthead-generated light field, particularly adjustment instructions relating to a change in one or more features of a lighthead-generated light field, around the lighthead-generated light field, by means of at least one laser light source included therein and by means of a light-transmitting optical film by conducting the light of said laser light source through the optical film toward a surgical site on the patient to be illuminated.

The adjustment instructions refer in this context to adjustment instructions relating to changes in a feature of the lighthead-generated light field. The adjustment instructions pertain to the operation of a lighthead's user input elements, i.e. are explicitly operating instructions for user input elements.

The adjustment instructions consist preferably of adjustment instruction patterns pertinent to features of a lighthead's light field adjustable or generable with user input elements.

The user input elements refer in this context for example to a switch, a button or a touch-sensitive area whose flipping, pressing or touching enables selecting or adjusting a controllable feature of the lighting system's light field, especially that of a lighthead.

The adjustment instructions may refer to features pertinent to a specific switch, button or touch-sensitive area of the user input elements or how to operate said switch, button or touch-sensitive area of the user input elements for modifying the lighthead-generated light field.

The adjustment instruction pattern(s) are regarded in this context as pictograms, logograms, and words presentable in visual form.

The adjustment instruction is preferably produced in the immediate vicinity of a lighthead-generated light field, i.e. immediately around the light field. Especially in the event that an adjustment instruction is produced immediately around the light field, an exact location of the adjustment instruction will be determined, still depending on an embodiment, optionally on the basis of a mutual location and/or orientation (position) of the projection elements and the laser light source.

Depending on the embodiment, the invention provides several benefits with respect to the prior art. When the adjustment instructions are projected in a light field or its surroundings, the operator of a lighthead is able to conveniently adjust a desired feature without taking his/her eyes off an illuminated target such as, in medical context, e.g. off a patient undergoing surgery or examination or his/her tissue while simultaneously operating the adjustment elements of a lighthead's user interface. Thus, for example, changing the position of a lighthead operator's head does not lead to the necessity of adjusting the lighting several times for the reason that the lighting is adjusted incorrectly as the effect of a head shadow cannot be taken effectively into consideration when adjusting the lighting.

In particular, when the adjustment instructions are projected around a light field, there is also attained a benefit of the projected adjustment instructions not falling for example upon a surgical site. Hence, the procedure, for example a surgical operation, is not disturbed in any way by the presentation of adjustment instructions either, as the lighthead's light field is intended to be focused on a site of the patient subjected to a medical procedure and as the adjustment instructions are projected around this light field. It is the optics employed in projecting which determine where the adjustment instructions are projected and which can be handily arranged around a light field in such a way that for example the surgeon need not turn his/her head during a surgical operation in order to see the adjustment instructions.

The invention comprises projecting adjustment instructions pertinent to adjustment elements, such as user input elements, for controlling features of a light field generated by a lighthead or generable by a lighthead.

The adjustment elements can be used not only for adjusting each selected or currently "active" feature as indicated by instructions, such as by turning the elements or a component thereof, e.g. a handle, in a specific direction (which can be indicated by an arrow), but also for selecting a feature subjected to adjustment from among a plurality of features.

Most preferably, the selection of a feature and the actual adjustment can be managed without essentially removing or repositioning one's hand from the lighthead's user interface between the discussed procedures. This can be achieved by positioning the user input elements for selection and adjustment procedures in the proximity of each other, for example in the form of a touch-sensitive pushbutton or the like tactile area and a rotatable handle or switch.

The operator may also assure him-/herself about an active feature to be adjusted at a given time by identifying adjustment instructions, which are visible at that particular moment and which have been designed in the best possible way to be inherently associable with the discussed feature. For example, the adjustment instructions for color temperature may include a symbol 'K', whereby the skilled person knows immediately that an adjustment of color temperature (K->Kelvin, unit for color temperature) is in question.

Providing adjustment instructions by using a special separate light source or sources, such as semiconductor lasers, enables a projection of the instructions as sharply as well as distinctly as possible and thereby in a manner easy to read and absorb for the operator e.g. even in conditions of bright background lighting.

At least in certain embodiments, the production of instructions can be carried out by utilizing fixed optics by virtue of so-called simplicity and reliability. Alternatively, the system may include e.g. translatorily and/or rotatably movable elements such as a mask, lens, mirror, or the like, thus providing necessary variation in projecting.

In some embodiments, optionally those of fixed optics, different instructions can be implemented by means of different light sources. E.g. the light of a first laser can be used for producing first instructions and the light of a second laser for second instructions. The lasers' light beams fall preferably on different points in the optics of projection elements, such as in a film which is light transmitting yet includes a optical light-controlling or -modulating surface and/or embedded structures

By projecting adjustment instructions for a light field feature as a repeating pattern or structure, e.g. in the form of a circle, capable of being read or identified from various directions, the operator is readily able to read the instructions from various angles and positions without essentially shifting his/her location or position from the original, possibly optimal working position or location.

Other benefits gained by embodiments of the present invention will be introduced subsequently in this disclosure.

The terms "first", "second" and "third" are used for distinguishing elements from each other. As a rule, the terms do not express the order, number or significance between the elements unless explicitly mentioned otherwise in the disclosure.

The term "a number of" is used in this disclosure in reference to any positive integer such as the number 1, 2 or 3, while the term "a plurality of" refers to any positive integer starting from number 2, such as to the number 2, 3, 4 or 5.

Various embodiments of the invention will be described in more detail hereinafter in a specific segment of the specification as well as in dependent claims.

### BRIEF DESCRIPTION OF THE FIGURES

Embodiments of the invention will now be described with reference to the accompanying drawings, in which
Fig. 1 shows axonometrically a lighthead according to one embodiment of the invention.
Fig. 2 shows one embodiment of adjustment elements according to the invention and particularly of a user interface of the adjustment elements for modifying light field features.
Fig. 3 shows one embodiment relating to a lighthead layout of light sources adapted to spotlighting and light sources adapted to projecting adjustment instructions.
Fig. 4 shows a lighthead according to one embodiment of the inventive lighthead in a working situation, wherein the light field generated on a site to be illuminated has its rim provided with projected adjustment instructions for modifying a feature of the light field.
Fig. 5 shows an alignment/aiming pattern generated, preferably along with adjustment instructions, by one lighthead embodiment of the invention.
Fig. 6 shows one embodiment for first projectable adjustment instructions.
Fig. 7 shows one embodiment for second projectable adjustment instructions.
Fig. 8 shows one embodiment for third projectable adjustment instructions.
Fig. 9 is a flowchart for one embodiment of a method according to the invention.
Fig. 10 is a combined structural and block diagram concerning selected features of one lighthead embodiment of the invention

### DETAILED DESCRIPTION

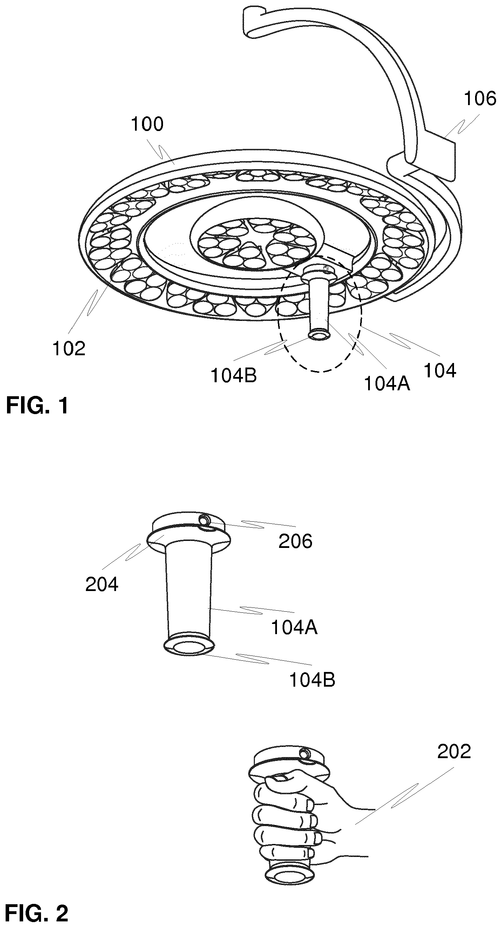

Fig. 1 shows a lighthead 100 according to one embodiment of the invention.

The lighthead 100 comprises a plurality of light sources 102, preferably LED lamps, which are preferably physically and functionally disposed in groups capable of being separately controlled, such as switched on and off. The light sources or the groups of light sources may have been additionally configured for a so-called circular form to thereby make up two rings, a so-called inner ring and outer ring, of light sources or groups of light sources (as in the figure).

The lighthead 100 has a user interface 104 for modifying light field features. The user interface 104 includes preferably a plurality of user input elements, such as for example a handle 104A and a selector 104B, optionally a capacitive or resistive contact switch or contact area optionally positioned at an end of the handle 104A or the like element. The selector 104B may have been constructed e.g. by mounting or printing, preferably by means of printable electronics (additively, e.g. silk-screen, flexography or inkjet), on a support such as a plastic sheet or film, or some other article comprising an insulating material, an electrode comprising a conductive material or e.g. two possibly overlapping or superimposed electrodes. The electrodes, and if necessary the support thereof, may have been embedded within the structure or protected with a surface structure such as a sheet or shell e.g. for minimizing the risk of damage and for isolating (e.g. hermetically) the electrodes from a working environment. By monitoring capacitance between electrodes or e.g. capacitance between an electrode and an external element, there is achieved an ability to detect, by means of fairly simple observation electronics/circuit, an external element such as a finger in the proximity of a particular electrode or electrodes.

The lighthead 100 can have its frame attached by way of a connecting member 106 to external structures, such as to the ceiling structures of a surrounding space, e.g. an operating room, examination room or workspace, or optionally e.g. to a stand or other support equipped with wheels.

The preferably removable, sterilized and/or antibacterial handle 104A, which is connected to the frame of the lighthead 100, enables features of the lighthead's light field to be modified in a beneficial manner. For example, an essentially translatory motion applied to the handle can be used for changing the light field of the lighthead 100 in terms of its orientation. Hence, in the lighthead 100, such as especially in its above-mentioned connecting member 106, can be included one or more articulations, thus enabling the frame element comprising the light sources 102 to be directed by moving, i.e. turning, this element from the handle 104A notwithstanding a possibly fixed installation of the lighthead 100 and particularly its connecting member 106.

On the other hand, the handle 104A is preferably capable of being turned (rotated) by the operator around its own longitudinal axis. By turning the handle 104A, the operator is able to optionally adjust a light field feature such as brightness (as implemented e.g. by means of the intensity of light sources and technically by way of power control), color temperature, or area of illumination (e.g. size, diameter and/or shape). A first turning direction of the handle 104A can be linked with increasing a feature, such as the value of color temperature (e.g. a numerical value in Kelvins), and a second, opposite turning direction respectively with decreasing.

It is by turning the handle 104A that the feature to be adjusted at a given time can be preferably chosen by means of the selector 104B. For example, the selector 104B can be configured to interpret a user input function of the selector 104B, such as pressing or touching, so as to replace a current adjustable feature with the next one in a predetermined sequence of features. The sequence may also include a condition in which the adjustment is not in a so-called on-state (i.e. turning of the handle 104A) does not make any difference in light field features).

Additionally or alternatively, the duration of a user input procedure, such as the duration of a contact applied to a touch-sensitive surface, can be registered and, depending on the duration, the input can be configured to trigger e.g. a start and/or termination for the display of adjustment measures and/or adjustment instructions, optionally in an alternating manner, or some other function.

Fig. 2 shows one user interface embodiment for modifying light field features. The user interface can be utilized e.g. in connection with the lighthead embodiment of fig. 1.

The user interface handle 104A can be removably connectable, preferably without tools, to the rest of the lighthead 100, such as to its frame, as presented in fig. 1. A fixed position of the handle 104a can be mechanically ensured e.g. with the assistance of a spring mechanism, a clip and/or magnets; the handle or its counterpart in the lighthead 100 can be configured to include e.g. a pushbutton, a lever or some other user-operable element 206 for momentarily overcoming a spring force or other force holding the handle 104a fixedly in the lighthead 100 and for releasing the handle 104A from its counterpart. The handle 104A may further include a protrusion or a lip 204 or some other formation, which prevents the user's hand from inadvertently contacting the discussed release mechanism and which assists in placing a hand 202 in its intended location and remaining in its proper place.

The user may be able to operate the handle 104A and thereby for example to adjust light field features with his/her hand 202 as shown in the figure or in some other way, optionally by holding his/her thumb on the selector 104B and other fingers around the handle 104A.

It is possible that the handle 104A has its turning mechanism fitted e.g. with an increasing turn resistance creating element, such as a (returning) spring, by means of which the risk of unintended adjustment measures can be reduced. In addition to or instead of this, the turning mechanism can be provided with serrations, which indicate to the user operating the handle 104A that an adjustment command has reached its destination (in other words, as rotation of the handle 104A passes or reaches a serration point, the adjustment command becomes registered by the lighthead).

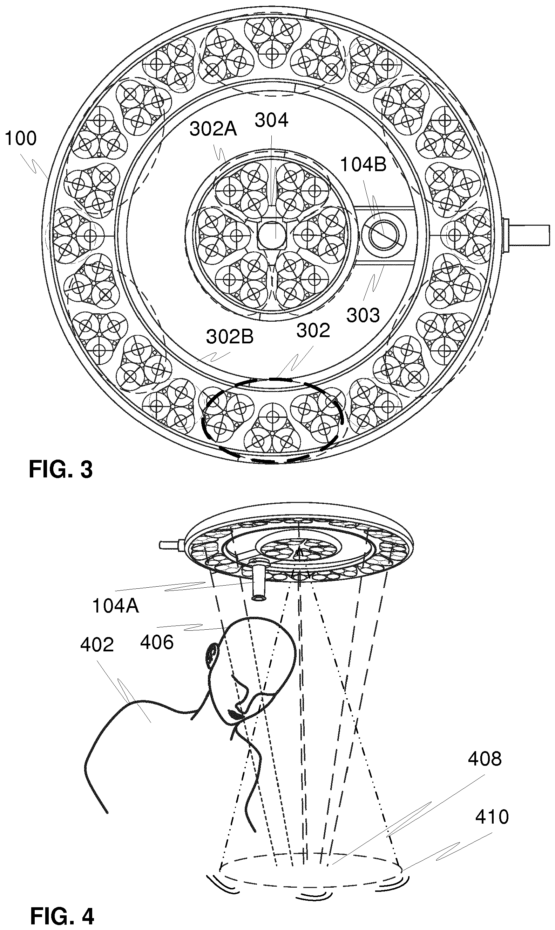

Fig. 3 shows a lighthead implementation according to one embodiment, regarding the layout of light sources, such as LED light sources, adapted for general illumination of a target area, and one or more light sources adapted for producing adjustment instructions, with respect to the rest of the lighthead. In other words, the question is about a plan view of the lighthead as seen from below or more generally from the lighting direction.

The light sources 102 are configured functionally and also structurally into discrete groups 302 thereof optionally symmetrically relative to the midpoint or center axis of a pattern constituted by the light source groups 302. At least one light source 304 capable of being utilized in projecting adjustment instructions, such as a laser light source, or a structure out-coupling the light produced by the light source 304, including elements guiding the lights, such as a mirror, is positioned essentially in alignment with a midpoint/axis 304 or more generally in the middle of other light sources 102 or light source groups 302 e.g. with respect to an average propagation direction of the light.

As pointed out above, the light sources, and the light source groups 302 including the same, may have been specifically disposed in a so-called inner ring formation 302A and an outer ring formation 302B, both of which are supported by their specific, e.g. circular or annular frame sections. The sections can be connected to each other by means of at least one intermediate section 303, in which intermediate section 303 can in turn be disposed e.g., user interface elements such as the handle 104A and the selector 104B preferably as shown in the figure to be accessible to the user.

Fig. 4 shows the operation of one lighthead embodiment, such as any of the embodiments shown in figs. 1-3, in a working situation, wherein the rim of a light field 408 generated on a site of illumination is provided with projected adjustment instructions 410 for modifying a feature of the light field by means of lighthead adjustment elements and in response to a user input received by way of the user interface 104 included in the adjustment elements, such as by way of the handle 104A. A user 402 is therefore able to keep his/her eyes on a target area or on a so-called site of operation without taking glances during the adjustment toward switches or the like of the lighthead's 100 user interface.

In the illustrated scenario, some of the lighthead-emitted light beams fall at a point 406 (which is highlighted by means of changing dashed lines) upon the back of the user's 402 head, therefore not migrating all the way to a target and thereby not contributing in a desired manner to a light field at the target. Indeed, in some lighthead embodiments of the invention, it is possible to utilize e.g., a distance sensor (e.g. infrared or ultrasound transmitters and receivers) to detect possible obstacles in an optical transmission pathway, such as in the air, between lighthead and target. When an obstacle is detected between some light sources and a target, the obstacle's adverse effect on illumination of the target and on this particular light field can be compensated for by means of other light sources e.g. by activating replacement light source groups from the lighthead.

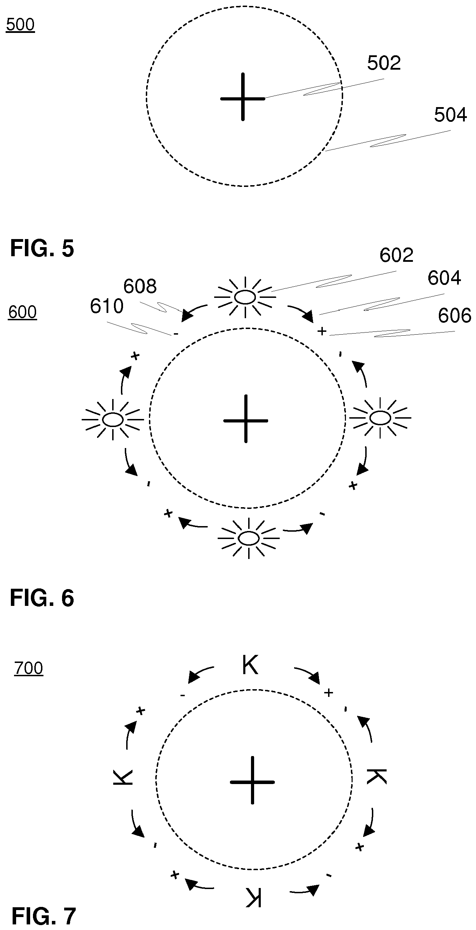

Fig. 5 shows, at 500, a focusing/aiming pattern produced by one lighthead embodiment of the invention, such as a point or more preferably an easier-to-detect, more complex shaped and/or larger pattern such as a cross 502. In addition, the pattern may but need not include e.g. a circumferential, such as circular indicator 504, such as a line or dashed line, which demonstrates e.g. an essential size/diameter and location (e.g. the edge with respect to a boundary value selected for the lighting intensity) of the light field.

It is by means of the pattern 502, 504 that the orientation of a light field is easy to ensure and, if necessary, to change as desired e.g. by placing the cross 502 on top of the midpoint of a site of surgical operation.

Fig. 6 shows at 600 one embodiment for first projectable adjustment instructions. A sun symbol 602 can be used for indicating to the user that e.g. a brightness adjustment is in question. Technically, the (perceived) brightness of a target can be increased by means of the lighthead 100 e.g. by increasing the volume of a light stream generated by the light sources 102.

In relation to the adjustment of e.g. said brightness but also other features, a '+' symbol 606 and a curved arrow 604 pointing thereto indicate that the adjustable feature, such as brightness, increases by turning an element, such as the handle 104A, of the user interface 104 for adjustment elements in the designated direction.

A '-' symbol 610 and a curved arrow 608 pointing thereto indicate that the adjustable feature decreases by turning an element, such as the handle 104A, of the user interface 104 for adjustment devices in the designated (opposite) direction.

Fig. 7 shows at 700 one embodiment for second projectable adjustment instructions, which preferably differ from the first instructions in terms of e.g. projected patterns, their size, location, brightness, color or the like. The adjustment of a light field feature relating to the second instructions, such as color temperature, takes place by utilizing preferably the same elements of the user interface 104, such as the handle 104A.

Between adjustments of features such as brightness 600 and temperature 700 can be alternated by means of the user interface 104, preferably by means of selector such as a push or contact switch comprising implemented touch sensitive area 104B.

In various embodiments of the invention, the so-called same adjustment instruction or a part thereof (referring e.g. to a symbol 'K' in fig. 7) can be presented preferably at the same time in a variety of ways, such as in different orientations, in order to make the instructions as easily understandable as possible in various working situations and from the perspective of various users, including various orientations and locations of a user or users, which are possibly changing even in one and the same working situation in a workspace.

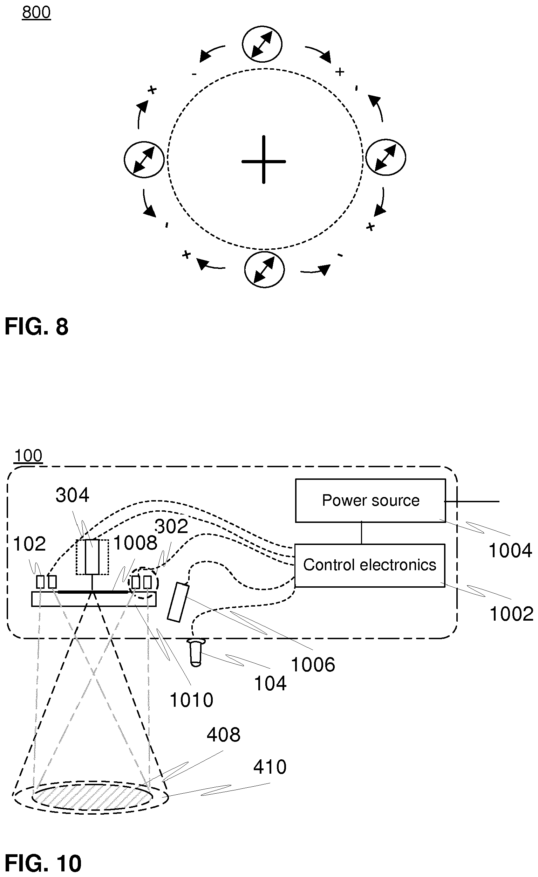

Fig. 8 shows at 800 one embodiment for third projectable adjustment instructions relating e.g. to the adjustment of an area of illumination, such as the adjustment of a size, diameter and/or shape. The same way as 'K' (Kelvin) in the previous adjustment instruction is demonstrative and easy to understand for a skilled person as a symbol of color temperature adjustment, the double-headed arrow inside a circle is likewise respectively highly suitable for the demonstration of adjustment relating to the dimensions of an area. Of course, instead of or in addition to symbols, the adjustable feature could be visualized in projection e.g. by means of text and/or numerals.

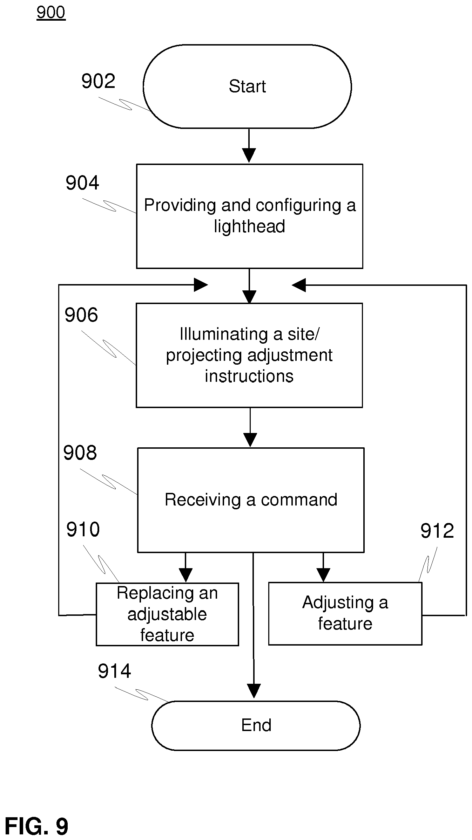

Fig. 9 includes a flowchart 900 for one embodiment of a method according to the invention.

Step 902 may comprise determining the requirements imposed on the illumination of and a lighthead for a target facility, such as an operating room, (medical) examination room or e.g. a product development or repair facility to enable selection of a lighthead that fulfills the requirements and has correct features

Step 904 comprises providing and configuring a lighthead according to any of the inventive embodiments. It can be mounted, among other things, to the ceiling structures of an operating room or other target facility, optionally to be movable e.g. along rails.

In some embodiments, the lighthead may be configured by a user, a fitter or installer or some other person in terms of lighthead functions such as adjustable features and/or adjustment interval. E.g. the sequence of adjustable features can be modifiable with regard to features included therein and/or the order thereof, especially if the adjustable feature is chosen by making use of a user input device, such as the selector 104B, which is common thereto. Configuration can be conducted by means of the lighthead's user interface or e.g. a separate maintenance interface.

Step 906 comprises illuminating an object, such as a patient or a device under construction and/or repair. The adjustment instructions are projected, preferably as described above, on the boundaries of a light field generated by spot lighting. The spot lighting is implemented according to adjustment values effective at the particular moment, such as e.g. brightness and color temperature.

Step 908 comprises receiving a command from the user by way of the lighthead's user input element such as the selector 104B or the handle 104A. The command can be an adjustment-related command such as a replacement/selection command for an adjustable (active) light field feature or an adjustment command for a feature already in active state, typically an adjustment value increasing or decreasing command. The user interface may comprise a number of user input elements also for purposes other than for switching the lighthead (or lighting) on and off.

When the command is an adjustment command, there is executed at step 912 the adjustment of an adjustable feature in response to the command. For example, turning the handle 104A for a certain time and/or to a certain extent may represent a change in the adjustment value of a feature in proportion to the time and/or the extent of turning the handle (rotation angle). Alternatively, a single rotating action may accomplish a preselected change in the adjustment value regardless of the time or the extent of rotation.

When the command is a replacement/selection command received by way of user input elements such as the selector 104B, there is executed at step 910 the replacement of each feature, which is adjustable through a user interface by means of user input elements such as the handle 104A, on the basis of a selection logic programmed in the lighthead by proceeding, e.g. in the adjustment sequence, from a current feature (e.g. color temperature) to the next (e.g. brightness).

Execution of the method is brought to an end at point 914. This point can be reached e.g. when the user terminates operation of the lighthead by giving through the user interface a shutdown command to the lighthead, by utilizing e.g. a shutdown switch/button.

The feedback arrows illustrated in the figure indicate a possible continuous/repeating nature of practicing the method. During a single working cycle, it is possible to adjust various light field features several times by an operator of the lighthead.

Fig. 10 is a combined structural and block diagram regarding selected features for one embodiment of a lighthead 100 according to the invention.

The lighthead 100 comprises a film 1008, which is preferably permeable to light at its desired wavelengths such as within the range of visible light and which is optically essentially transparent (e.g. light transmission of at least 80%, 85% or 90%). The film can be e.g. a flexible plastic film such as a polymer film. The film may contain e.g. polycarbonate or PMMA (polymethylmethacrylate).

The film 1008 is optically functional. It can be adapted to produce an adjustment instruction pattern from light generated by at least one light source 304. For the purpose, the film 1008 may contain refractive and/or diffractive surface-relief structures e.g. in micrometer level size class or even smaller and/or (subsurface) structures e.g. laminated or otherwise embedded in a multilayer structure. These light-conducting and/or -modulating structures may comprise lattice grooves, other recesses, ridges, bulges, etc. In cross-section, the structures may comprise various sharp or rounder shapes such as triangular shapes, parallelogram shapes, rectangular shapes, trapezoidal shapes, etc. The film 1008 may contain a number of optical masks.

For each adjustment instruction pattern the film 1008 may contain a number of dedicated light-conducting/modulating structures or e.g. structure groups including several structures positioned physically in the proximity of each other.

Optionally, the film 1008 or a respective separate film can be adapted to conduct, modulate or at least to allow light through itself also from other light sources 102 or light source groups 302.

The film 1008 can be e.g. less than 2mm, preferably less than 1mm or 0.5mm in thickness.

In connection with the film 1008 can be disposed, optionally cast e.g. by injection molding with the film 1008 serving as an insert, or laminated e.g. by means of pressure, an adhesive and/or pressure, a sheet protecting the optics 102, 302, 304 of the lighthead 100 and preferably comprising an optically essentially transparent material, such as a plastic or glass sheet 1010. The sheet 1010 can have a scratch-resistant, moisture-repellent and/or antibacterial surface (coating).

In some embodiments, at least some of the light sources 102, 302, 304 can be at least partially embedded in the film 1008 or in the sheet 1010 for protecting the sources and/or for improving optical coupling between themselves and the film/sheet 1008, 1010.

In some embodiments, the element 1010 may contain other optics or provide a so-called optical functionality, such as functioning at least locally as a lens.

The employed light sources 102 can be surface mount components and/or produced with printed electronics methods, e.g. by inkjet printing or silk-screen printing with reference e.g. to OLED sources (organic LED).

The light source 304 for presenting adjustment instructions may include a number or a plurality of laser light sources such as semiconductor lasers. These light sources 304 can be explicitly adapted to the presentation of adjustment instructions. The possible presence of several light sources 304, optionally disposed adjacent to or otherwise in the proximity of each other, is depicted in the figure in dashed lines at the element 304.

In the case of several light sources 304, an individual light source can be dedicated (exclusively adapted) to project some specific adjustment instruction pattern. Respectively, in order to project this particular pattern, the film 1008 may contain dedicated optical structures as indeed pointed out above.

Control electronics 1002 may comprise a microcircuit, a microprocessor, a microcontroller, a programmable logic or other control unit for controlling the light sources 102, 304, the groups 302, and optionally other elements such as a sensor 1006 and/or for receiving commands therefrom (cf. the user interface 104 including e.g. the handle 104A). A power source 1004 may comprise power electronics for energizing the lighthead's control electronics 1002 and other elements such as the light sources 102. The power source 1004 can be connected e.g. by way of a cable to an external power supply, such as to a stage of the general power grid.

The elements to be controlled may also comprise movable elements, such as translatorily and/or rotatably movable elements. These elements can be optical, such as masks, lenses and/or mirrors, by means of which the optical transmission pathway between the light sources 102, 304 and an object can be modified.

The lighthead 100 contains also a plurality of other elements, not shown in the figure for the sake of clarity. These elements include e.g. a frame, articulation parts, connecting parts for attachment e.g. to ceiling structures or other support members. The elements may comprise e.g. plastics or metal. Likewise, e.g. the handle 104A and also other user interface elements (e.g. those brought often to human contact) may contain plastics such as polysulfone (PSU), which is preferably capable of withstanding e.g. numerous sterilization cycles.

The element 1006 refers to one or more sensors, such as a distance sensor as mentioned above, which the lighthead 100 may include e.g. for detecting obstacles in an optical transmission pathway between the lighthead 100 and an object. Upon detecting an obstacle, the light sources 102 or the light source groups 302 can be controlled respectively, most simply by switching on or off, to compensate for a difference made by the obstacle in illumination of the object. Optionally the sensor 1006 can be used for measuring a distance between the lighthead 100 and an object, on the basis of which e.g. the intensity of lighting can be adjusted optionally so as to keep it constant.

## Claims

1. An operating room lighthead (100), said lighthead comprising a plurality of light sources (102) for generating a light field (408) on a site of surgical operation to be illuminated on a patient, as well as adjustment elements (1002) for modifying features of said light field, said adjustment elements comprising a user interface for modifying features of the light field by the actions of a lighthead operator (104), **characterized in that** the user interface further comprises projection elements (1008) for projecting, around the light field, adjustment instructions (5) related to a feature of the lighthead-generated light field, and the lighthead is further provided with at least one laser light source (304) specifically for projecting said adjustment instructions, and the projection elements comprise a light-transmitting optical film (1008) which is adapted to produce an adjustment instruction from light generated by said at least one laser light source (304), said film comprising diffractive and/or refractive optical microstructures for producing adjustment instructions.

2. A lighthead according to claim 1, wherein the plurality of light sources are LED elements.

3. A lighthead according to any of the preceding claims, wherein the at least one laser light source (304) is a semiconductor laser.

4. A lighthead according to any of the preceding claims, wherein the diffractive and/or refractive optical microstructures are surface-relief structures and/or embedded structures

5. A lighthead according to any of the preceding claims, wherein the location of adjustment instructions around a light field is determined on the basis of a mutual location of at least the projection elements and the laser light source.

6. A lighthead according to any of the preceding claims, wherein the optical film has on its surface a protective layer, such as a sheet or a glass or plastic layer.

7. A lighthead according to any of the preceding claims, wherein the adjustment instructions (5) are operating instructions, which relate to user input elements and which consist of an adjustment instruction pattern pertaining to light field features adjustable or achievable by means of the user input elements.

8. A lighthead according to any of the preceding claims, which is adapted to project the adjustment instructions dynamically based on a light field feature adjustable at a particular time and selected by the user.

9. A lighthead according to any of the preceding claims, which is adapted to project, for at least two adjustable light field features, mutually different adjustment instructions (600, 700, 800).

10. A lighthead according to any of the preceding claims, wherein the projected adjustment instructions comprise graphic patterns or symbols (602, 604, 606, 608, 610).

11. A lighthead according to any of the preceding claims, which is adapted to project the adjustment instructions as a regularly repeating pattern or structure on or around the light field.

12. A lighthead according to any of the preceding claims, wherein said at least one light source comprises two light sources, the first of which is adapted to generate the light needed in the projection of first adjustment instructions and the second is adapted to generate the light needed in the projection of second adjustment instructions.

13. A lighthead according to claim 9, which is adapted to activate a first or second light source producing adjustment instructions for a modifiable first or second feature of a light field generated by the user-selected lighthead indicated by way of a user interface.

14. A lighthead according to any of the preceding claims, wherein said film is preferably a flexible polymer film.

15. A lighthead according to any of the preceding claims, wherein said film is less than 2mm, preferably less than 1mm, and most preferably less than 0.5mm in thickness.

16. A lighthead according to any of the preceding claims, wherein said adjustable feature of a lighthead-generated light field comprises at least one light field feature selected from the group of: brightness, color temperature, diameter, shape and size.

17. A lighthead according to any of the preceding claims, wherein said user interface includes optionally an antibacterial and/or sterilized adjustment handle or roller (104A), by rotation of which some feature of a lighthead-generated light field is selectable or adjustable, respectively.

18. A lighthead according to claim 17, wherein said user interface includes one or more user input elements (104B), preferably a switch, button or touch-sensitive area, by turning, pressing, or touching of which some adjustable feature of the lighthead-generated light field is selectable or adjustable, respectively.

19. A lighthead according to claim 18, said user interface of which includes a touch-sensitive capacitive user input element (104B).

20. A lighthead according to any of the preceding claims, wherein said projection elements include a preferably translatorily and/or rotatably movable, optionally motorized, optical element such as a mask, lens, or mirror for selecting at each time presentable adjustment instructions by way of a user interface on the basis of some user-indicated modifiable feature of the lighthead-generated light filed.

21. A method (900) for presenting illumination adjustment instructions to the operator of an operating room lighthead, said lighthead including a plurality of light sources for generating a light field on a surgical site on the patient, as well as adjustment elements for modifying features of said light field, said adjustment elements being provided with a user interface for modifying features of the lighthead-generated light field by the actions of a lighthead operator, **characterized in that** the method comprises
projecting (906) adjustment instructions for modifying features of the lighthead-generated light field, particularly adjustment instructions relating to a change in one or more features of a lighthead-generated light field, around the lighthead-generated light field, by means of at least one laser light source (304) included therein and by means of a light-transmitting optical film (1008) by conducting the light of said laser light source through the optical film toward a surgical site in the patient to be illuminated.

22. A method according to claim 21, wherein the light sources are LED elements.

23. A method according to claim 21 or 22, wherein the at least one laser light source (304) is a semiconductor laser.

24. The method of any of the preceding claims 21-23, wherein the optical film is a diffractive and/or refractive film.

25. A method according to any of the preceding claims 21-24, wherein projecting takes place dynamically based on some each time adjustable, user-selected feature of a lighthead-generated light field.

26. A method according to any of the preceding claims 21-25, which comprises projecting for at least two adjustable light field features mutually different adjustment instructions (600, 700, 800) for the change of a light field feature.

27. A method according to any of the preceding claims 21-26, which comprises distinguishing adjustment instructions for a first adjustable feature from adjustment instructions for a second adjustable feature by using at least one distinction practice for distinguishing the instructions visually from each other, said practice having been selected from the group of: a graphic pattern or symbol, color, size, location and brightness.

28. A method according to any of the preceding claims 21-27, which comprises producing the adjustment instructions for a first adjustable feature by means of a first light source, preferably by a first laser such as a semiconductor laser, and producing the adjustment instructions for a second adjustable feature by means of a second light source, preferably a second laser.

29. A method according to any of the preceding claims 21-28, which comprises, in response to the selection of an adjustable light field feature performed by the user through a user interface, projecting the adjustment instructions, which relate to said feature and are visually different from adjustment instructions relating to at least one other adjustable feature.

## Patentansprüche

1. Operationssaallampenkopf (100), wobei der Lampenkopf mehrere Lichtquellen (102) zum Erzeugen eines Lichtfelds (408) auf einer Operationsstelle, die an einem Patienten beleuchtet werden soll, sowie Einstellungselemente (1002) zum Modifizieren von Merkmalen des Lichtfelds umfasst, wobei die Einstellungselemente eine Benutzeroberfläche zum Modifizieren von Merkmalen des Lichtfelds durch die Handlungen eines Lampenkopfbedieners (104) umfassen, **dadurch gekennzeichnet, dass** die Benutzeroberfläche ferner Projektionselemente (1008) zum Projizieren, um das Lichtfeld herum, von Einstellungsanweisungen (5) umfasst, die ein Merkmal des durch den Lampenkopf erzeugten Lichtfelds betreffen, und der Lampenkopf ferner mit zumindest einer Laserlichtquelle (304) spezifisch zum Projizieren der Einstellungsanweisungen versehen ist, und die Projektionselemente eine lichtdurchlässige optische Folie (1008) umfassen, die zum Erzeugen einer Einstellungsanweisung aus Licht, welches durch die zumindest eine Laserlichtquelle (304) erzeugt wird, geeignet ist, wobei die Folie diffraktive und/oder refraktive optische Mikrostrukturen zum Erzeugen von Einstellungsanweisungen umfasst.

2. Lampenkopf nach Anspruch 1, wobei die mehreren Lichtquellen LED-Elemente sind.

3. Lampenkopf nach einem der vorhergehenden Ansprüche, wobei die zumindest eine Laserlichtquelle (304) ein Halbleiterlaser ist.

4. Lampenkopf nach einem der vorhergehenden Ansprüche, wobei die diffraktiven und/oder refraktiven optischen Mikrostrukturen Oberflächenreliefstrukturen und/oder eingelassene Strukturen sind.

5. Lampenkopf nach einem der vorhergehenden Ansprüche, wobei die Stelle von Einstellungsanweisungen um ein Lichtfeld auf der Basis einer wechselseitigen Stelle von zumindest den Projektionselementen und der Laserlichtquelle bestimmt wird.

6. Lampenkopf nach einem der vorhergehenden Ansprüche, wobei die optische Folie auf ihrer Oberfläche eine Schutzschicht aufweist, wie etwa eine Platte oder eine Glas- oder Kunststoffschicht.

7. Lampenkopf nach einem der vorhergehenden Ansprüche, wobei die Einstellungsanweisungen (5) Bedienungsanweisungen sind, die mit Benutzereingabeelemente zusammenhängen und aus einem Einstellungsanweisungsmuster bestehen, welche zu Lichtfeldmerkmalen gehören, die mithilfe der Benutzereingabeelemente einstellbar oder erzielbar sind.

8. Lampenkopf nach einem der vorhergehenden Ansprüche, der dazu geeignet ist, die Einstellungsanweisungen basierend auf einem Lichtfeldmerkmal, das zu einer bestimmten Zeit einstellbar ist und durch den Benutzer ausgewählt wird, dynamisch zu projizieren.

9. Lampenkopf nach einem der vorhergehenden Ansprüche, der dazu geeignet ist, für zumindest zwei einstellbare Lichtfeldmerkmale wechselseitig unterschiedliche Einstellungsanweisungen (600, 700, 800) zu projizieren.

10. Lampenkopf nach einem der vorhergehenden Ansprüche, wobei die projizierten Einstellungsanweisungen grafische Muster oder Zeichen (602, 604, 606, 608, 610) umfassen.

11. Lampenkopf nach einem der vorhergehenden Ansprüche, der dazu geeignet ist, die Einstellungsanweisungen als ein sich regelmäßig wiederholendes Muster oder Struktur auf oder um das Lichtfeld herum zu projizieren.

12. Lampenkopf nach einem der vorhergehenden Ansprüche, wobei die zumindest eine Lichtquelle zwei Lichtquellen umfasst, wobei deren erste dazu geeignet ist, das Licht zu erzeugen, das bei der Projektion von ersten Einstellungsanweisungen benötigt wird, und die zweite dazu geeignet ist, das Licht zu erzeugen, das bei der Projektion von zweiten Einstellungsanweisungen benötigt wird.

13. Lampenkopf nach Anspruch 9, der dazu geeignet ist, eine erste oder zweite Lichtquelle zu aktivieren, welche Einstellungsanweisungen für ein modifizierbares erstes oder zweites Merkmal eines Lichtfelds erzeugt, das durch den vom Benutzer ausgewählten Lampenkopf erzeugt wird, der mittels einer Benutzeroberfläche angezeigt wird.

14. Lampenkopf nach einem der vorhergehenden Ansprüche, wobei die Folie vorzugsweise eine flexible Polymerfolie ist.

15. Lampenkopf nach einem der vorhergehenden Ansprüche, wobei die Folie eine Stärke von weniger als 2 mm, vorzugsweise weniger als 1 mm und meist bevorzugt weniger als 0,5 mm aufweist.

16. Lampenkopf nach einem der vorhergehenden Ansprüche, wobei das einstellbare Merkmal eines vom Lampenkopf erzeugten Lichtfelds zumindest ein Lichtfeldmerkmal umfasst, das aus der Gruppe von: Helligkeit, Farbtemperatur, Durchmesser, Form und Größe ausgewählt ist.

17. Lampenkopf nach einem der vorhergehenden Ansprüche, wobei die Benutzeroberfläche optional eine/n antibakterielle/n und/oder sterilisierte/n Griff oder Rolle (104A) enthält, durch dessen/deren Drehung ein Merkmal eines durch den Lampenkopf erzeugten Lichtfelds auswählbar bzw. einstellbar ist.

18. Lampenkopf nach Anspruch 17, wobei die Benutzeroberfläche ein oder mehr Benutzereingabeelemente (104B), vorzugsweise einen Schalter, Knopf oder berührungsempfindlichen Bereich, enthält, durch deren Umlegen, Drücken oder Berühren ein einstellbares Merkmal des durch den Lampenkopf erzeugten Lichtfelds auswählbar bzw. einstellbar ist.

19. Lampenkopf nach Anspruch 18, dessen Benutzeroberfläche ein berührungsempfindliches kapazitives Benutzereingabeelement (104B) enthält.

20. Lampenkopf nach einem der vorhergehenden Ansprüche, wobei die Projektionselemente ein vorzugsweise translatorisch und/oder drehend bewegliches, optional motorisiertes, optisches Element wie etwa eine Maske, eine Linse oder einen Spiegel zum Auswählen von zu jeder Zeit präsentierbaren Einstellungsanweisungen mittels einer Benutzeroberfläche auf der Basis eines vom Benutzer angezeigten, modifizierbaren Merkmals des durch den Lampenkopf erzeugten Lichtfelds enthalten.

21. Verfahren (900) zur Präsentation von Beleuchtungseinstellungsanweisungen an einen Bediener eines Operationssaallampenkopfs, wobei der Lampenkopf mehrere Lichtquellen zum Erzeugen eines Lichtfelds auf einer Operationsstelle, die an einem Patienten beleuchtet werden soll, sowie Einstellungselemente zum Modifizieren von Merkmalen des Lichtfelds umfasst, wobei die Einstellungselemente mit einer Benutzeroberfläche zum Modifizieren von Merkmalen des Lichtfelds durch die Handlungen eines Lampenkopfbedieners versehen sind, **dadurch gekennzeichnet, dass** das Verfahren umfasst:
Projizieren (906) von Einstellungsanweisungen zum Modifizieren von Merkmalen des durch den Lampenkopf erzeugten Lichtfelds, insbesondere Einstellungsanweisungen bezüglich einer Veränderung bei einem oder mehr Merkmalen eines durch den Lampenkopf erzeugten Lichtfelds, mithilfe von zumindest einer Laserlichtquelle (304), die darin enthalten ist, und mithilfe einer lichtdurchlässigen optischen Folie (1008) durch Leiten des Lichts der Laserlichtquelle durch die optische Folie zu einer Operationsstelle an einem Patienten, die beleuchtet werden soll.

22. Verfahren nach Anspruch 21, wobei die Lichtquellen LED-Elemente sind.

23. Verfahren nach Anspruch 21 oder 22, wobei die zumindest eine Laserlichtquelle (304) ein Halbleiterlaser ist.

24. Verfahren nach einem der vorhergehenden Ansprüche 21 bis 23, wobei die optische Folie eine diffraktive und/oder refraktive Folie ist.

25. Verfahren nach einem der vorhergehenden Ansprüche 21 bis 24, wobei das Projizieren basierend auf einem jedes Mal einstellbaren, vom Benutzer ausgewählten Merkmal eines durch den Lampenkopf erzeugten Lichtfelds dynamisch stattfindet.

26. Verfahren nach einem der vorhergehenden Ansprüche 21 bis 25, das das Projizieren, für zumindest zwei einstellbare Lichtfeldmerkmale, wechselseitig unterschiedliche Einstellungsanweisungen (600, 700, 800) für die Veränderung eines Lichtfeldmerkmals umfasst.

27. Verfahren nach einem der vorhergehenden Ansprüche 21 bis 26, das das Unterscheiden von Einstellungsanweisungen für ein erstes einstellbares Merkmal von Einstellungsanweisungen für ein zweites einstellbares Merkmal durch Verwenden von zumindest einer Unterscheidungspraxis zum visuellen Unterscheiden der Anweisungen voneinander umfasst, wobei die Praxis aus der Gruppe von: einem grafischen Muster oder Symbol, Farbe, Größe, Lage und Helligkeit ausgewählt wurde.

28. Verfahren nach einem der vorhergehenden Ansprüche 21 bis 27, das das Erzeugen der Einstellungsanweisungen für ein erstes einstellbares Merkmal mithilfe einer ersten Lichtquelle, vorzugsweise einem ersten Laser, wie etwa einem Halbleiterlaser, und das Erzeugen der Einstellungsanweisungen für ein zweites einstellbares Merkmal mithilfe einer zweiten Lichtquelle, vorzugsweise einem zweiten Laser, umfasst.

29. Verfahren nach einem der vorhergehenden Ansprüche 21 bis 28, das, in Reaktion auf die Auswahl eines einstellbaren Lichtfeldmerkmals, welche durch den Benutzer über eine Benutzeroberfläche ausgeführt wird, das Projizieren der Einstellungsanweisungen, die sich auf das Merkmal beziehen und sich visuell von Einstellungsanweisungen unterscheiden, die sich auf zumindest ein anderes einstellbares Merkmal beziehen, umfasst.

## Revendications

1. Projecteur de salle d'opération (100), ledit projecteur comprenant une variété de sources lumineuses (102) pour générer un champ lumineux (408) sur un site d'opération chirurgicale à illuminer sur un patient, ainsi que des éléments d'ajustement (1002) pour modifier les caractéristiques dudit champ lumineux, lesdits éléments d'ajustement comprenant une interface d'utilisateur pour modifier des caractéristiques du champ lumineux par les actions d'un opérateur de projecteur (104), **caractérisé en ce que** l'interface d'utilisateur comprend en outre des éléments de projection (1008) pour projeter autour du champ lumineux des instructions d'ajustement (5) relatives à une caractéristique du champ lumineux généré par le projecteur, et le projecteur est en outre pourvu d'au moins une source de lumière laser (304) spécifiquement pour projeter lesdites instructions d'ajustement, et les éléments de projection comprennent un film optique transmettant la lumière (1008) qui est adapté pour produire une instruction d'ajustement à partir de la lumière générée par ladite au moins une source de lumière laser (304), ledit film comprenant des microstructures optiques diffractives et/ou réfractives pour produire des instructions d'ajustement.

2. Projecteur selon la revendication 1, dans lequel la pluralité de source lumineuse sont des éléments DEL.

3. Projecteur selon une quelconque des revendications précédentes, dans lequel au moins une source de lumière laser (304) est un laser à semi-conducteurs.

4. Projecteur selon une quelconque des revendications précédentes, dans lequel les microstructures optiques diffractives et/ou réfractives sont des structures à relief de surface et/ou des structures incorporées.

5. Projecteur selon une quelconque des revendications précédentes, dans lequel l'emplacement des instructions d'ajustement autour d'un champ lumineux est déterminé sur la base d'un emplacement réciproque d'au moins les éléments de projection et la source de lumière laser.

6. Projecteur selon une quelconque des revendications précédentes, dans lequel le film optique possède sur sa surface une couche protectrice, comme un film ou une couche de verre ou de plastique.

7. Projecteur selon une quelconque des revendications précédentes, dans lequel les instructions d'ajustement (5) sont des instructions de fonctionnement, qui se rapportent aux éléments de saisie par l'utilisateur et qui sont constituées d'un modèle d'instruction d'ajustement se rapportant aux caractéristiques de champ lumineux pouvant être ajustées obtenues au moyen des éléments de saisie par l'utilisateur.

8. Projecteur selon une quelconque des revendications précédentes, qui est adapté pour projeter les instructions d'agissement dynamiquement sur la base d'une caractéristique de champ lumineux pouvant être ajustée à un moment particulier et sélectionnée par l'utilisateur.

9. Projecteur selon une quelconque des revendications précédentes, qui est adapté pour projeter, pour au moins deux caractéristiques de champ lumineux ajustables, des instructions d'ajustement réciproquement différentes (600,700,800).

10. Projecteur selon une quelconque des revendications précédentes, dans lequel les instructions d'ajustement projetées comprennent des motifs ou des symboles graphiques (602,604,606,608,610).

11. Projecteur selon une quelconque des revendications précédentes, qui est adapté pour projeter les instructions d'ajustement comme un motif ou une structure se répétant régulièrement sur ou autour du champ lumineux.

12. Projecteur selon une quelconque des revendications précédentes, dans lequel ladite au moins une source lumineuse comprend deux sources lumineuses, dont la première est adaptée pour générer la lumière nécessaire pour la projection des premières instructions d'ajustement et la seconde est adaptée pour générer la lumière nécessaire pour la projection des secondes instructions d'ajustement.

13. Projecteur selon la revendication 9, qui est adaptée pour activer une première seconde source lumineuse produisant des instructions d'ajustement pour une première ou une seconde caractéristique modifiable d'un champ lumineux généré par le projecteur sélectionné par l'utilisateur indiqué au moyen d'une interface d'utilisateur.

14. Projecteur selon une quelconque des revendications précédentes, dans lequel ledit film est de préférence un film polymère flexible.

15. Projecteur selon une quelconque des revendications précédentes, dans lequel ledit film est inférieur à 2 mm, de préférence inférieur à 1 mm, et de manière la plus préférée inférieur à 0.5 mm en épaisseur.

16. Projecteur selon une quelconque des revendications précédentes, dans lequel ladite caractéristique ajustable d'un champ lumineux généré par le projecteur comprend au moins une caractéristique de champ lumineux sélectionnée dans le groupe composé de : luminosité, température de couleur, diamètre, forme et taille.

17. Projecteur selon une quelconque des revendications précédentes, dans lequel ladite interface d'utilisateur inclut optionnellement une poignée ou un galet de réglage (104A) antibactérien et/ou stérilisé, par la rotation duquel certaines caractéristiques ajustables d'un champ lumineux généré par le projecteur peuvent être sélectionnées ou ajustées, respectivement.

18. Projecteur selon la revendication 17, dans lequel ladite interface d'utilisateur inclut un ou plusieurs éléments de saisie par l'utilisateur (104B), de préférence un interrupteur, un bouton ou une zone tactile sensible, en tournant, pressant ou touchant lequel certaines caractéristiques ajustables du champ lumineux généré par le projecteur peuvent être sélectionnées ou ajustées, respectivement.

19. Projecteur selon la revendication 18, dont l'interface d'utilisateur inclut un élément de saisie par l'utilisateur capacitif tactile (104B).

20. Projecteur selon une quelconque des revendications précédentes, dans lequel lesdits éléments de projection incluent un élément optique déplaçable en translation et/ou rotation, optionnellement motorisé, tel qu'un masque, une lentille ou un miroir pour sélectionner à chaque moment des instructions d'ajustement pouvant être présentées au moyen d'une interface d'utilisateur sur la base de certaines fonctionnalités modifiables indiquées par l'utilisateur du champ lumineux généré par le projecteur.

21. Procédé (900) pour présenter des instructions d'ajustement d'illumination à l'opérateur d'un projecteur de salle d'opération, ledit projecteur incluant une pluralité de sources lumineuses pour générer un champ lumineux sur un site chirurgical sur le patient, ainsi que des éléments d'ajustement pour modifier les caractéristique dudit champ lumineux, lesdits éléments d'ajustement étant fournis par une interface d'utilisateur pour modifier des fonctionnalités du champ lumineux généré par le projecteur par les actions d'un opérateur de projecteur, **caractérisé en ce que** le procédé comprend de
projeter (906) des instructions d'ajustement pour modifier les caractéristiques du champ lumineux généré par le projecteur, notamment des instructions d'ajustement relatives à un changement d'une ou plusieurs caractéristiques d'un champ lumineux généré par le projeteur, autour du champ lumineux généré par le projecteur, au moyen d'au moins une source de lumière laser (304) incluse dans ce dernier est au moyen d'un film optique transmettant la lumière (1008) en conduisant la lumière de ladite source de lumière laser à travers le film optique dans la direction d'un site chirurgical sur le patient à illuminer.

22. Procédé selon la revendication 21, dans lequel les sources lumineuses sont des éléments DEL.

23. Procédé selon la revendication 21 ou 22, dans lequel au moins une source de lumière laser (304) est un laser à semi-conducteurs.

24. Procédé selon une quelconque des revendications précédentes 21-23, dans lequel le film optique est un film diffractif et/ou réfractif.

25. Procédé selon une quelconque des revendications précédentes 21-24, dans lequel la projection a lieu de manière dynamique sur la base d'une fonctionnalité sélectionnée par l'utilisateur ajustable à tout moment d'un champ lumineux généré par le projecteur.

26. Procédé selon une quelconque des revendications précédentes 21-25, qui comprend de projeter pour au moins deux caractéristiques de champ de lumière ajustables des instructions d'ajustement mutuellement différentes (600,700,800) pour le changement d'une caractéristique de champ lumineux.

27. Procédé selon une quelconque des revendications précédentes 21-26, qui comprend de distinguer des instructions d'ajustement pour une première caractéristique ajustable des instructions d'ajustement pour une seconde caractéristique ajustable en utilisant au moins une pratique de distinction pour distinguer les instructions visuellement les unes des autres, ladite pratique ayant été sélectionnée dans le groupe de : un motif ou un symbole graphique, une couleur, une taille de un emplacement et une luminosité.

28. Procédé selon une quelconque des revendications précédentes 21-27, pour une première caractéristique ajustable au moyen d'une première source lumineuse, de préférence par un premier laser tel qu'un laser à semi-conducteurs et de produire les instructions d'ajustement pour une seconde caractéristique ajustable au moyen d'une seconde source lumineuse, de préférence un second laser.

29. Procédé selon une quelconque des revendications précédentes 21-28, qui comprend, en réponse à la sélection d'une fonctionnalité de champ lumineux ajustable effectuée par l'utilisateur par l'intermédiaire d'une interface d'utilisateur, de projeter les instructions d'ajustement, qui se rapportent à ladite caractéristique et sont visuellement différente des instructions d'ajustement relatives à au moins une autre caractéristique ajustable.
